# EUROPEAN PATENT SPECIFICATION

(11) **EP 4 501 290 B1**
(45) Date of publication and mention of the grant of the patent: **29.04.2026**
(21) Application number: 24190979.5
(22) Date of filing: 25.07.2024
(51) Int. Cl.: A61F 5/44, A61F 5/451

(54) **RECEPTACLE AND SYSTEMS FOR HUMAN WASTE COLLECTION AND CONTAINMENT**
BEHÄLTER UND SYSTEME ZUM SAMMELN UND EINSCHLIESSEN VON MENSCHLICHEN ABFÄLLEN
RÉCEPTACLE ET SYSTÈMES DE COLLECTE ET DE CONFINEMENT DE DÉCHETS HUMAINS

(30) Priority: 02.08.2023 US 202318363992
(43) Date of publication of application: 05.02.2025
(73) Proprietor: Hamilton Sundstrand Space Systems International, Inc., Windsor Locks, CT 06096 (US)
(72) Inventor: LUKER, Kelly, Glastonbury, CT 06033 (US); RADAWIEC, Rochelle, Vernon, CT 06066 (US)
(74) Representative: Dehns

(56) References cited:
- US-A- 3 329 974
- US-A- 4 360 933
- US-A- 5 894 608

## Description

### BACKGROUND

The subject matter disclosed herein generally relates to systems and mechanisms for collection and containment of human waste and, more particularly, to improved funnel and collection devices related thereto.

When humans reside in relatively closed spaces or environments (e.g., on ships, aircraft, spacecraft, or the like), or in remote locations or the like, human waste must be managed to avoid contamination, disease, sanitation, and the like. If conventional plumbing is not required or there are environmental impacts to plumbing functionality, specific or uniquely designed systems may be required to manage human waste. For example, when in low, micro, or no gravity (e.g., in space), a lack of gravity prevents conventional plumbing from being employed. Further, due to the low or no gravity, containment of liquids and solids is an important consideration.

In current systems, a hose with a funnel is connected to a waste collection system and a fan or the like is used to generate a suction force. A user (e.g., human user) will apply the funnel to the body and excrete urine or menses into the funnel, which captures the waste. The connected hose will provide a path through which the waste is pulled (or pushed) and the liquid and gaseous portions may be separated, with the liquid (and/or any solids) may be directed into a holding container or the like. Current systems were designed primarily based on male anatomy, and thus the current systems are lacking for fit and use by females. For example, current funnels on universal waste management systems are not adequately fitted for females and as such, female users can experience backsplash when urinating. Urinating can also be messy because the urine flow is not always a directed velocity into an airstream used to collect and capture the liquid. The addition of menses waste can create similar non-optimal effects. Although current funnels may be usable and functional, the funnels must be replaced on a regular basis and treated as a consumable component. Accordingly, improved systems for a broader range of use, for improved capture, direction, and containment of human waste, and/or for increased product/device life may be advantageous in a variety of applications. A waste collection system is disclosed in US 5 894 608 A.

### SUMMARY

According to the claimed invention, waste management systems are provided as defined by claim 1.

Embodiments of the waste management systems may include that the funnel comprises at least one airflow aperture arranged to permit gases to pass therethrough and prevent liquid from passing through the at least one airflow aperture.

Embodiments of the waste management systems may include that the skirt is adjustable between a minimum deployment and a maximum deployment.

Embodiments of the waste management systems may include that the skirt is formed from silicone, a silicone blend, or a flexible, anti-microbial material.

Embodiments of the waste management systems may include that the skirt has the same non-circular shape as the inlet end of the funnel.

Embodiments of the waste management systems may include that the non-circular shape is a teardrop shape or a saddle shape.

Embodiments of the waste management systems may include that the capture insert is formed from a wicking material.

Embodiments of the waste management systems may include that the capture insert if formed from a hydrophobic material, a hydrophilic material, or a combination thereof.

Embodiments of the waste management systems may include that the capture funnel comprises a hose adapter at the outlet end of the funnel, wherein the hose adapter is configured to sealing connect to the hose.

Embodiments of the waste management systems may include a fluid driver arranged along the hose and configured to generate a motive force through the hose and through the capture funnel.

Embodiments of the waste management systems may include a fluid separation junction arranged along the hose and configured to separate liquid and gas fluids passing through the hose.

Embodiments of the waste management systems may include that the waste container comprises a removable container insert.

Embodiments of the waste management systems may include that the waste management system is configured for use in a low gravity environment.

The foregoing features and elements may be combined in various combinations without exclusivity, unless expressly indicated otherwise. These features and elements as well as the operation thereof will become more apparent in light of the following description and the accompanying drawings. It should be understood, however, that the following description and drawings are intended to be illustrative and explanatory in nature and non-limiting and the scope of the invention is as defined by the claims.

### BRIEF DESCRIPTION OF THE DRAWINGS

The subject matter is particularly pointed out and distinctly claimed at the conclusion of the specification. The foregoing and other features, and advantages of the present disclosure are apparent from the following detailed description taken in conjunction with the accompanying drawings in which:
FIG. 1 is a schematic illustration of a waste management system in accordance with an embodiment of the present disclosure;
FIG. 2 is a schematic illustration of a capture funnel for use with a waste management system in accordance with an embodiment of the present disclosure;
FIG. 3A is a schematic illustration of a capture funnel of the present disclosure shown in a minimum deployment state;
FIG. 3B is a schematic illustration of a capture funnel of the present disclosure shown in an intermediate deployment state;
FIG. 3C is a schematic illustration of a capture funnel of the present disclosure shown in a maximum deployment state; and
FIG. 4 is a cross-sectional illustration of a capture funnel in accordance with an embodiment of the present disclosure.

### DETAILED DESCRIPTION

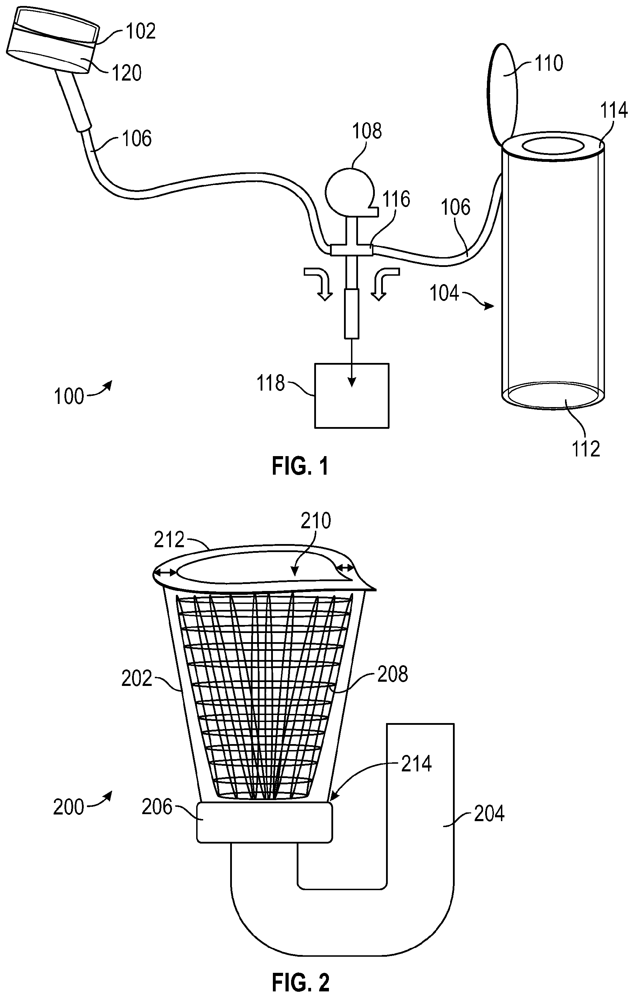

Referring to FIG. 1, a schematic diagram of a waste management system 100 in accordance with an embodiment of the present disclosure is shown. The waste management system 100 may be used onboard a ship, craft, or in a remote location. The specific described embodiments and examples of this disclosure are directed to use onboard a spacecraft or the like that is subject to low gravity, microgravity, or no gravity, when in space. However, it will be appreciated that the waste management system 100 and variations thereon, and as described herein, may be employed in other environments and/or situations, and the present description is not intended to be limited to space-based (low gravity) applications. As used herein, the term "low gravity" will be used to refer to low gravity, microgravity, and no gravity situations collectively, for ease of use and description.

The waste management system 100 includes a capture funnel 102 that is fluidly coupled to a waste container 104 by a hose 106 (e.g., hose, tube, conduit, etc.). The capture funnel 102 is configured and arranged to be applied by a user directly on the skin or with an intermediate layer (e.g., cloth layer or the like). In use, the capture funnel 102 will direct human waste (e.g., urine and/or menses) toward and into the hose 106. The hose 106 may be configured with a fluid driver 108 (e.g., pump, fan, or the like) that creates air suction and aids in directing the liquid waste into and through the hose 106. The liquid (and any solid) waste may be directed along the hose 106 to the waste container 104 and/or a reclamation system 118. For example, in some configurations, by operation of the fluid driver 108, liquid waste may be pulled from the waste container 104 and directed into the reclamation system 118.

In accordance with some embodiments, the waste container 104 may be a selectively resealable container that includes a sealable lid 110 and a removable container insert 112. The waste container 104 may be configured to receive both liquid and solid human waste. In some configurations, as noted above, the fluid driver 108 may be used to extract liquid waste from the waste container 104 and direct it to the reclamation system 118. By removal of liquid from the waste container 104, solid waste may remain for disposal using the removable container insert 112. The removable container insert 112 may be secured in the waste container 104 by the sealable lid 110 engaging with a rim 114 of the waste container 104. When the removable container insert 112 is indicated as full, the removable container insert 112 may be removed and disposed of or emptied, and a new or clean (emptied) removable container insert 112 may be installed back into the waste container 104. It will be appreciated that other configurations are possible without departing from the scope of the present disclosure. For example, in some configurations, the removable container insert 112 may not be present, and the waste container 104 may be configured to be emptied directly and sanitized, for example.

As shown, a fluid separation junction 116 may be provided along the hose 106 between the capture funnel 102 and the waste container 104. The fluid separation junction 116 may be configured to separate liquid and gaseous portions of a fluid passing through the hose 106. For example, in some configurations, a source of clean air (e.g., cabin air) may be directed into and through the capture funnel 102 and/or the hose 106 to aid in driving the fluid away from a user and toward the reclamation system 118. As shown, downstream from the fluid separation junction 116 and separate from the waste container 104 is the reclamation system 118. The reclamation system 118 may include a holding tank or storage tank and various other components or systems for treating or reclaiming both gas and liquid from the waste management system 100 (e.g., directly from a user or from the waste container 104). For example, the reclamation system 118 may be configured to receive, separate, and treat liquid waste and gas (e.g., air) from the waste management system 100. In other non-limiting configurations, air (and/or other gases) is reclaimed and may also be used in a semi-closed system for use with the waste management system 100 by a subsequent user. It will be appreciated that such air may be used for other purposes, such as for cooling, treating, thermal exchange, or the like, in one or more other systems onboard the same craft as the waste management system 100.

The capture funnel 102 may be configured for use by a human user for capture and disposal of human waste (e.g., urine). In conventional systems, the capture funnel 102 is a generally uniform cup, or the like, that male users have an easier time "docking" into and urinating directly into the air stream. However, such configuration may not be as viable (e.g., useable, sanitary, etc.) when used by female users. Accordingly, in accordance with embodiments of the present disclosure, the capture funnel 102 may be designed and arranged to more properly fit to the female anatomy and thus provided improvements in use, sanitation, cleanliness, and the like. For example, in this illustrative configuration, the capture funnel 102 may include a capture insert 120 that can prevent splashing of liquid directed into the capture funnel 102. Further, in some embodiments, the shape, size, contour, and supporting features may be modified to provide further improvements in waste capture when used by female anatomy users and potentially by male anatomy users as well.

In accordance with a non-limiting example, the capture funnel 102 may be selectively removable from the hose 106. It will be appreciated that the waste management system 100 may be used for both liquid and solid waste disposal. As such, in some configurations, the capture funnel 102 and a section of the hose 106 associated therewith may be removable, leaving only a portion of the hose 106 connecting the waste container 104 to the reclamation system 118. Furthermore, in accordance with some configurations, each user of the waste management system 100 may provide their own capture funnel 102 that is selectively attachable to the hose 106. As such, a female user may use a first capture funnel 102 having features as described herein, and a male user may use a second capture funnel (not shown) that is substantially similar to conventional capture funnels. In conventional capture funnels, an open end of the capture funnel may be substantially circular in shape and elongated to accommodate male anatomy. This circular shape and potentially elongated shape may make female use more difficult, as the circular opening may not seal adequately to the skin of the female user. Accordingly, a more ergonomic or contoured open end may be employed in accordance with embodiments of the present disclosure to provide a more secure and complete seal between the open end of the capture funnel and the user (e.g., seal to skin of user) during use.

Furthermore, in accordance with some embodiments of the present disclosure, capture funnels described herein provide for a funnel with a wicking insert designed specifically for female bodies to more adequately direct urine into a urine hose of a waste management system (e.g., such as waste management system 100 of FIG. 1). In accordance with some embodiments described herein, a mouth of the capture funnel may be shaped to fit female anatomy. In such configurations, a capture insert may be positioned inside the funnel for urine droplets to attach to in a low gravity environment. The capture insert may be formed of a wicking material or the like (e.g., hydrophobic, hydrophilic, or a combination thereof depending on the specific application). In accordance with some embodiments, the material of the capture funnel and/or the capture insert may be anti-microbial and selected to withstand UV-light or other sanitation mechanisms (e.g., selected for use with specific sanitizing chemicals or the like).

In accordance with some embodiments of the present disclosure, a mouth or opening of the capture funnel may be fitted or shaped to fit to the female body. In some such embodiments, instead of a circular mouth or opening (e.g., for conventional or male-use versions), a contoured geometry or non-circular shape or geometry may be provided for female users for optimal comfort. The non-circular shapes may be teardrop, oval, elongate, or the like, as will be appreciated by those of skill in the art. Such contoured shaped capture funnels (or even the male versions) may include a capture insert that provides a wicking capability for capture of liquid directed into the capture funnel. For example, during use, urine may attach to the capture insert instead of freely floating around the capture funnel, and thus messes associated with free-floating liquid may be avoided. In accordance with some embodiments of the present disclosure, the capture funnel may be provided with an opening or mouth that is designed to mold or fit to the female anatomy via one or more expandable wings. Such winged version may be capable of fitting most or all female bodies (e.g., fit 5th and 95th percentile female crewmembers).

As noted, a capture insert within the capture funnel may provide wicking functionality to aid in capture and directing of fluids into and through the capture funnel and into and through a connected hose. In accordance with some embodiments, the capture insert can be built into the capture funnel as an affixed or permanent feature or, in some embodiments, may be a removable or disposable insert element. In some such embodiments, the capture insert may be selectively removable and configured to be treated and/or sanitized for extended life and use. That is, the capture insert may be a single-use consumable, a limited-use consumable, or reusable device, depending on material and other considerations. In some configurations, a UV sanitation locker may be provided to extend the life of the capture funnel and/or the capture insert. In some configurations, the capture funnel may be provided with an interface to lock into the hose of the waste management system, thus providing a fluidly sealed connection therebetween. Because the capture funnels may be user-specific (e.g., each user has their own capture funnel), the locking mechanism may be provided to ensure that each time a user connects their capture funnel to the hose, a properly sealed connection may be provided.

Referring now to FIG. 2, a schematic illustration of a capture funnel 200 in accordance with an embodiment of the present disclosure is shown. The capture funnel 200 may be configured for use with a waste management system, such as waste management system 100 shown in FIG. 1. For example, the capture funnel 200 of FIG. 2 may be used in place of the capture funnel 120 shown in FIG. 1. The capture funnel 200 is designed for use by a female user, and thus the arrangement, shape, and features may be designed specifically for the female anatomy. The capture funnel 200 includes a funnel 202 that is connectable to a hose 204 by a hose adapter 206. The hose adapter 206 may be part of and/or integrally formed with the funnel 202, may be part of and/or integrally formed with the hose 204, or may be a separate component that selectively and/or releasable attached to each of the hose 204 and the funnel 202. The hose adapter 206 is configured to provide a fluidly sealed connection between the funnel 202 and the hose 204.

In this illustrative configuration, the capture funnel 200 includes a capture insert 208 installed within the funnel 202. At an end opposite the hose adapter 206, the capture funnel 200 includes an inlet end 210 (e.g., open end). At the inlet end 210, the capture funnel 200 includes a skirt 212 that defines a rim or edge of the capture funnel 200 for contact and engagement with a user (e.g., skin of a user). The skirt 212 is an optional feature that may be provided to expand or increase the circumference or surface area of engagement between the inlet end 210 of the capture funnel 200 and the user. The skirt 212 may be adjustable in size (e.g., width) to increase or decrease an amount of material that is positioned in contact with a user.

The inlet end 210 and the skirt 212 may be shaped or contoured to fit snuggly and sealingly against a user. In accordance with some embodiments of the present disclosure, the inlet end 210 may have a substantially non-circular (e.g., teardrop, oval, elongated shape, etc.) shape which may conform to female anatomy more completely than a circular opening. The skirt 212 may have the same shape and contouring as the inlet end 210. The skirt 212 may be an adjustable element that can be increased in size and provide a sealing surface to engage with a user. For example, in some embodiments and without limitation, the skirt 212 may be a rubber, a soft plastic, silicone, a silicone blend, or other material that can provide a sealed and/or suction engagement or contact with skin of a user and thus provide a fluid seal between the skirt 212 and the user. The material for the skirt 212 may be a flexible material having anti-microbial properties. In some non-limiting embodiments, the skirt 212 may be a rolled configuration that is unrolled to a desired size for use and rolled up for storage. In other embodiments, the skirt 212 may be a folded configuration, which is unfolded for a desired size and folded for storage. In either of these configurations, or similar adjustable size configurations, the skirt 212 may be continuously adjustable such that there is a gradient of sizes. In other configurations, there may be preset sizes to which the skirt 212 may unroll/unfold. In other configurations, the skirt 212 may be extended and always present and provide a sealing engagement to a user, but may not be specifically adjustable between different sizes of contact. The skirt 212 provides for a wing-like structure or feature that provides sealing to a user. Such a seal can prevent splashing of liquid outside of the capture funnel 200. Further, depending on the specific waste management configuration, the seal provided by the skirt 212 can provide improved sealing engagement to prevent external air from being pulled into (or being blown out from) the funnel 202, thus improving sanitation of use of the capture funnel 200.

The capture funnel 200, in this embodiment, includes the capture insert 208. The capture insert 208 is an insert that is installed within the funnel 202 and may be arranged as a liner thereof. The funnel 202 may be substantially rigid, such as formed from a plastic or other rigid material which may be selected to be easily cleaned or sanitized. In some embodiments, the funnel 202 may be slightly flexible to allow a user to adjust the fit during use. However, in either instance, the material of the funnel 202 may be sufficient rigid or firm to cause splashing of liquid that is directed into the funnel 202. Additionally, if the material of the funnel 202 is not configured to absorb or capture liquid, in a low gravity environment, any liquid directed into the funnel 202 may become free floating and thus can result in a mess, release of such liquid droplets into an environment after use, or may otherwise not be pulled from the funnel 202 into the hose 204. The capture insert 208 is provided to reduce or eliminate these aspects of use and/or to provide additional functionality.

The capture insert 208 may be a single-use device or may be a multi-use device. In the case of a multi-use device, the capture insert 208 may be cleaned, sanitized, or treated between uses. The capture insert 208 may be formed from a wicking material that captures liquid droplets and prevent splash-back or free-floating droplets. As noted above, a fan or other airflow may be induced within the capture funnel 200 and/or the hose 204. In such configurations, the motive force provided by the airflow can drive the liquid toward the hose adapter 206 end of the capture funnel 200 (e.g., outlet end 214) and into the hose 204. The capture insert 208 is a mesh formed from fabric or the like. For example, and without limitation, the capture insert 208 may be made from cotton, natural fibers, acrylics, or other polymers made to be hydrophobic or hydrophilic.

In some embodiments, the outlet end 214 of the capture insert 208 may be open providing an open space through which liquid and gases may easily pass. In such a configuration, the capture insert 208 may be substantially cylindrical in shape and line sidewalls of the funnel 202 (e.g., conical in shape with an open tip/end). In other configurations, in addition to lining the sidewalls of the funnel 202, the capture insert 208 may have a continuous coverage at the outlet end 214 (e.g., conical in shape with a closed tip/end), with such end permitting the extraction of captured liquids to be drawn through the material of the capture insert 208 and pulled into the hose 204.

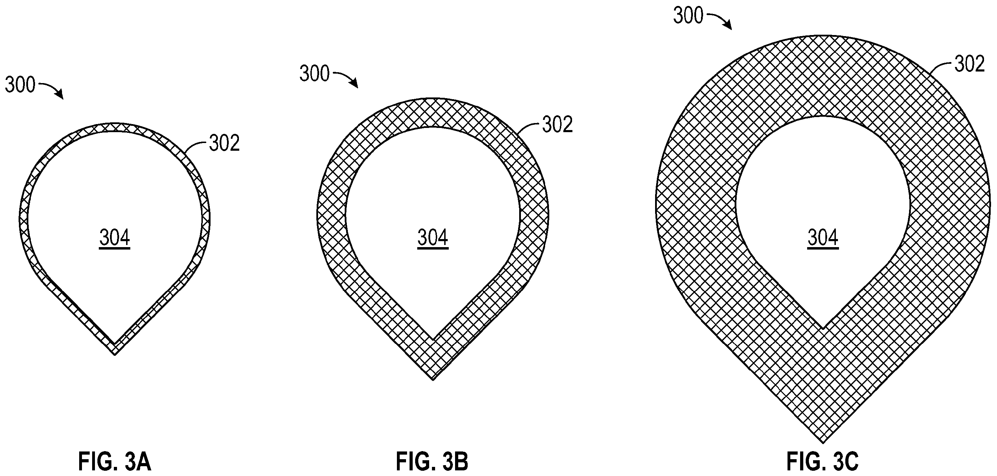

Referring now to FIGS. 3A-3C, schematic illustrations of different states of use of a capture funnel 300 in accordance with embodiments of the present disclosure are shown. The capture funnel 300 may be similar to the capture funnels shown and described above. The views of FIGS. 3A-3C are top-down views of the capture funnel 300. The capture funnel 300, as shown, includes a skirt 302 similar to that shown and described above. In FIG. 3A, the skirt 302 is shown in a completely stowed or closed state or in a minimum deployment state. If the skirt 302 is rollable, then FIG. 3A represents a fully rolled up state of the skirt 302. If the skirt 302 is foldable, then FIG. 3A represents a fully folded state of the skirt 302. Although in this configuration the skirt 302 is described as folded or rolled up (or otherwise at a minimum deployment) the capture funnel 300 is still useable, and the skirt 302, in this state will still provide a sealing engagement and contact with a user to provide the functionality described herein. FIG. 3B illustrates the skirt 302 in a partially deployed or intermediate deployment state, and FIG. 3C illustrates the skirt 302 in a fully deployed state or maximum deployment state. In each of the configurations of FIGS. 3B-3C, the skirt 302 is configured to provide a sealing engagement and contact with a user.

It will be appreciated that the illustrative arrangements shown in FIGS. 3A-3C are merely specific example states of deployment, and a gradual deployment of the skirt 302 is possible for any size between the completely stowed, closed, or minimum deployment state and the fully open, fully deployed, or maximum deployment state. Stated another way, there are many intermediate or partially deployed states that exist between the closed (FIG. 3A) and fully open (FIG. 3C) states. As such, the partial deployment state of FIG. 3B is merely intended to be illustrative of one of the many partial/intermediate deployment states. When a user intends to use the capture funnel 300, they may manually deploy the skirt 302 to a desired size to best-fit their body.

As shown in FIGS. 3A-3C, the capture funnel 300 includes an inlet end 304 that is teardrop shaped. The skirt 302 has the same shape and surrounds the inlet end 304. In accordance with some embodiments, the inlet end 304 may be rigidly defined by the nature of the associated funnel and thus is not considered adjustable. In such configurations, the skirt 302 provides for adjustability while allowing for a more generically used funnel and associated inlet end 304. The skirt 302 provides for flexibility and application to many different anatomies of users. In other embodiments, the funnel itself may be compressible and/or adjustable in shape (e.g., manually adjustable), and the skirt 302 can provide broader versatility to the available anatomies to which the capture funnel 300 can be applied.

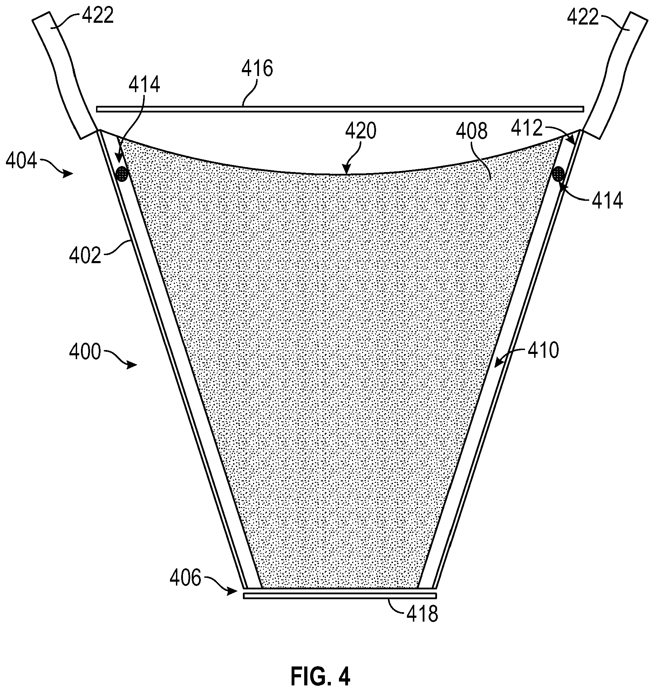

Referring now to FIG. 4, a cross-sectional schematic illustration of a capture funnel 400 in accordance with an embodiment of the present disclosure is shown. The capture funnel 400 may be similar to that shown and described above. The capture funnel 400 may include a funnel 402 that defines and extends between an inlet end 404 and an outlet end 406. The outlet end 406 may be configured to connect to a hose or hose connector, as shown and described above. The capture funnel 400, similar to that described above, includes a capture insert 408 installed within an interior of the funnel 402.

In this embodiment, an optional gap 410 is defined between the capture insert 408 and an interior surface 412 of the funnel 402. The gap 410 may provide a region or path for liquid to be directed toward the outlet end 406 of the funnel 402 while being prevented from splashing back or becoming free-floating. That is, liquid may be constrained within the gap 410. An airflow through the capture funnel 400 may provide a motive force to drive liquid toward the outlet end 406 of the funnel 402. Additionally, as shown, at least one airflow apertures 414 may be arranged about the funnel 402. In this illustrative embodiment, two airflow apertures 414 are provided proximate the inlet end 404 of the funnel 402. The airflow apertures 414 may be provided to allow for airflow to enter the capture funnel 400 and pass therethrough toward the outlet end 406 and thus provide a motive force for directing fluids (e.g., liquid droplets) through the capture funnel 400. In some embodiments, the airflow apertures 414 may be covered or filled with a material that allows for air to pass through but prevents liquid from passing therethrough. As such, liquid within the capture funnel 400 will not be permitted to exit through the airflow apertures 414.

The capture funnel 400, as shown in FIG. 4, includes an optional inlet cover 416 and an optional outlet cover 418. The covers 416, 418 may be selectively removable and installable on the respective ends 404, 406 of the funnel 402. The covers 416, 418 may be applied when the capture funnel 400 is not in use, and when a user intends to use the capture funnel 400, the covers 416, 418 may be removed. Accordingly, a clean, protected, and functional capture funnel for waste management systems is provided.

In accordance with some embodiments, and as shown in FIG. 4, the inlet end 404 of the capture funnel 400 may not be uniformly flat or even. That is, the open end (inlet end 404) may have a contoured opening 420, such as a saddle shape, that may be selected to improve contact and engagement between the inlet end 404 of the capture funnel 400 and a user. Such contoured opening 420 may be provided in combination with a skirt, such as shown and described above, thus further improving the usability for female users of the capture funnel 400. It will be appreciated that when viewing the capture funnel 400 from a side (e.g., left or right side relative to the view in FIG. 4), the top surface may be substantially flat due to the saddle shape (provided by contoured opening 420) of the inlet end 404. Further, although shown in FIG. 4 with the inlet cover 416 having a substantially flat shape, in other embodiments, the shape of the inlet cover may be similar or the same as the contoured opening 420 to provide improved sealing and protection to the capture funnel 400 when not in use. An optional harness 422 or the like may be provided with the capture funnel 400, as shown in FIG. 4. The harness 422 may be a set of adjustable straps, loops, elastic elements, or the like. The harness 422 can be attached at the inlet end 404 of the capture funnel 400 and a user can strap the harness 422 to their body and use the capture funnel 400 with no hands instead of requiring the capture funnel 400 to be held in place by hand. It will be appreciated that the harness 422 may also be used to ensure a complete seal and contact between the capture funnel 400 and the user's body.

Advantageously, embodiments described herein provide for improved waste management systems. In accordance with some embodiments of the present disclosure, waste management systems may be provided with improved capture funnels for use by users to reduce issues associated with waste capture. For example, in accordance with some embodiments, an improved contour shape of the capture funnels is provided to increase the usability and functionality for female users. The contour shape (e.g., teardrop) provides for a more ergonomic or fitted and customized geometry for receiving directed fluids (e.g., urine). Because of a lack of directionality, the capture funnel of some embodiments helps direct fluids to prevent splashing and droplet formation. Further such, geometry and contouring can provide for improved contact with a user to increase the sealing and preventing of leakage during and after use.

Furthermore, advantageously, capture inserts may be provided in the capture funnels to provide a wicking capability to capture and direct fluid through the capture funnel. The capture inserts may be formed from hydrophobic materials, hydrophilic materials, or combinations thereof to aid in capture and directing of fluids from an inlet end to an outlet end of the capture funnel and prevent splashing or free-floating droplet formation. The capture inserts are formed from a mesh or mesh-like material or a capillary-based material that can capture and direct fluid when in the presence of airflow or passively.

The use of the terms "a", "an", "the", and similar references in the context of description (especially in the context of the following claims) are to be construed to cover both the singular and the plural, unless otherwise indicated herein or specifically contradicted by context. The modifier "about" used in connection with a quantity is inclusive of the stated value and has the meaning dictated by the context (e.g., it includes the degree of error associated with measurement of the particular quantity). All ranges disclosed herein are inclusive of the endpoints, and the endpoints are independently combinable with each other.

While the present disclosure has been described in detail in connection with only a limited number of embodiments, it should be readily understood that the present disclosure is not limited to such disclosed embodiments. Rather, the present disclosure can be modified to incorporate any number of variations that fall within the scope of the invention as defined by the claims.

## Claims

1. A waste management system comprising:
a waste container (104);
a reclamation system (118);
a capture funnel (102); and
a hose (106) fluidly connecting the capture funnel to each of the waste container and the reclamation system,
wherein the capture funnel comprises:
a funnel (200) having an inlet end (210) and an outlet end (214), wherein the inlet end has a non-circular shape; and
a skirt (212) arranged at the inlet end; and **characterized by**:
a capture insert (120) installed within the funnel, wherein the capture insert comprises a mesh-like material or
a capillary-based material configured to capture liquid within a gap between the capture insert and an interior surface of the funnel to prevent the liquid from passing back through the capture insert and to direct the liquid toward the outlet end of the funnel.

2. The waste management system of claim 1, wherein the funnel comprises at least one airflow aperture arranged to permit gases to pass therethrough and prevent liquid from passing through the at least one airflow aperture.

3. The waste management system of claim 1 or 2, wherein the skirt (212) is adjustable between a minimum deployment and a maximum deployment.

4. The waste management system of any preceding claim, wherein the skirt is formed from silicone, a silicone blend, or a flexible, anti-microbial material.

5. The waste management system of any preceding claim, wherein the skirt has the same non-circular shape as the inlet end of the funnel, and optionally wherein the non-circular shape is a teardrop shape or a saddle shape.

6. The waste management system of any preceding claim, wherein the capture funnel comprises a hose adapter (206) at the outlet end of the funnel, wherein the hose adapter is configured to sealing connect to the hose.

7. The waste management system of any preceding claim, further comprising a fluid driver (108) arranged along the hose and configured to generate a motive force through the hose and through the capture funnel.

8. The waste management system of any preceding claim, further comprising a fluid separation junction (116) arranged along the hose and configured to separate liquid and gas fluids passing through the hose.

9. The waste management system of any preceding claim, wherein the waste container comprises a removable container insert (112).

10. The waste management system of any preceding claim, wherein the waste management system is configured for use in a low gravity environment.

## Patentansprüche

1. Abfallmanagementsystem, umfassend:
einen Abfallbehälter (104);
ein Rückgewinnungssystem (118);
einen Auffangtrichter (102); und
einen Schlauch (106), der den Auffangtrichter fluidisch mit jedem von dem Abfallbehälter und dem Rückgewinnungssystem verbindet,
wobei der Auffangtrichter Folgendes umfasst:
einen Trichter (200) mit einem Einlassende (210) und einem Auslassende (214), wobei das Einlassende eine nicht kreisförmige Form aufweist; und
eine Schürze (212), die am Einlassende angeordnet ist; und **gekennzeichnet durch**:
einen Auffangeinsatz (120), der innerhalb des Trichters installiert ist, wobei der Auffangeinsatz ein netzartiges Material oder ein auf Kapillarität basierendes Material umfasst,
das dafür konfiguriert ist, Flüssigkeit innerhalb eines Spalts zwischen dem Auffangeinsatz und einer Innenfläche des Trichters aufzufangen, um zu verhindern, dass die Flüssigkeit durch den Auffangeinsatz zurückfließt, und um die Flüssigkeit in Richtung des Auslassendes des Trichters zu leiten.

2. Abfallmanagementsystem nach Anspruch 1, wobei der Trichter mindestens eine Luftstromöffnung umfasst, die so angeordnet ist, dass Gase dort hindurchströmen können und verhindert wird, dass Flüssigkeit durch die mindestens eine Luftstromöffnung strömt.

3. Abfallmanagementsystem nach Anspruch 1 oder 2, wobei die Schürze (212) zwischen einer minimalen Entfaltung und einer maximalen Entfaltung einstellbar ist.

4. Abfallmanagementsystem nach einem der vorhergehenden Ansprüche, wobei die Schürze aus Silikon, einer Silikonmischung oder einem flexiblen, antimikrobiellen Material gebildet ist.

5. Abfallmanagementsystem nach einem der vorhergehenden Ansprüche, wobei die Schürze die gleiche nicht kreisförmige Form wie das Einlassende des Trichters aufweist und wobei die nicht kreisförmige Form gegebenenfalls eine Tropfenform oder eine Sattelform ist.

6. Abfallmanagementsystem nach einem der vorhergehenden Ansprüche, wobei der Auffangtrichter einen Schlauchadapter (206) am Auslassende des Trichters umfasst, wobei der Schlauchadapter dafür konfiguriert ist, abdichtend mit dem Schlauch verbunden zu werden.

7. Abfallmanagementsystem nach einem der vorhergehenden Ansprüche, ferner umfassend einen Fluidantrieb (108), der entlang des Schlauchs angeordnet und dafür konfiguriert ist, eine Antriebskraft durch den Schlauch und durch den Auffangtrichter zu erzeugen.

8. Abfallmanagementsystem nach einem der vorhergehenden Ansprüche, ferner umfassend eine Fluidtrennstelle (116), die entlang des Schlauchs angeordnet und dafür konfiguriert ist, flüssige und gasförmige Fluide zu trennen, die durch den Schlauch strömen.

9. Abfallmanagementsystem nach einem der vorhergehenden Ansprüche, wobei der Abfallbehälter einen entfernbaren Behältereinsatz (112) umfasst.

10. Abfallmanagementsystem nach einem der vorhergehenden Ansprüche, wobei das Abfallmanagementsystem zur Verwendung in einer Umgebung mit geringer Schwerkraft konfiguriert ist.

## Revendications

1. Système de gestion de déchets comprenant :
un conteneur à déchets (104) ;
un système de récupération (118) ;
un entonnoir de captation (102) ; et
un tuyau (106) reliant de manière fluidique l'entonnoir de captation à chacun du conteneur à déchets et du système de récupération,
dans lequel l'entonnoir de captation comprend :
un entonnoir (200) ayant une extrémité d'entrée (210) et une extrémité de sortie (214), dans lequel l'extrémité d'entrée a une forme non circulaire ; et
une jupe (212) disposée à l'extrémité d'entrée ; et **caractérisé par** :
un insert de captation (120) installé à l'intérieur de l'entonnoir, dans lequel l'insert de captation comprend un matériau de type maillé ou un matériau à base capillaire configuré pour capter le liquide à l'intérieur d'un espace entre l'insert de captation et une surface intérieure de l'entonnoir pour empêcher le liquide de repasser à travers l'insert de captation et pour diriger le liquide vers l'extrémité de sortie de l'entonnoir.

2. Système de gestion de déchets selon la revendication 1, dans lequel l'entonnoir comprend au moins une ouverture de flux d'air agencée pour permettre aux gaz de le traverser et empêcher le liquide de passer à travers l'au moins une ouverture de flux d'air.

3. Système de gestion de déchets selon la revendication 1 ou 2, dans lequel la jupe (212) est réglable entre un déploiement minimum et un déploiement maximum.

4. Système de gestion de déchets selon une quelconque revendication précédente, dans lequel la jupe est formée de silicone, d'un mélange de silicone ou d'un matériau flexible et antimicrobien.

5. Système de gestion de déchets selon une quelconque revendication précédente, dans lequel la jupe a la même forme non circulaire que l'extrémité d'entrée de l'entonnoir, et, éventuellement, dans lequel la forme non circulaire est une forme de larme ou une forme de selle.

6. Système de gestion de déchets selon une quelconque revendication précédente, dans lequel l'entonnoir de captation comprend un adaptateur de tuyau (206) au niveau de l'extrémité de sortie de l'entonnoir, dans lequel l'adaptateur de tuyau est configuré pour se relier de manière étanche au tuyau.

7. Système de gestion de déchets selon une quelconque revendication précédente, comprenant en outre un dispositif d'entraînement de fluide (108) agencé le long du tuyau et configuré pour générer une force motrice à travers le tuyau et à travers l'entonnoir de captation.

8. Système de gestion de déchets selon une quelconque revendication précédente, comprenant en outre une jonction de séparation de fluide (116) agencée le long du tuyau et configurée pour séparer les fluides liquides et gazeux passant à travers le tuyau.

9. Système de gestion de déchets selon une quelconque revendication précédente, dans lequel le conteneur à déchets comprend un insert de conteneur amovible (112).

10. Système de gestion de déchets selon une quelconque revendication précédente, dans lequel le système de gestion de déchets est configuré pour être utilisé dans un environnement à faible gravité.
